Europäisches Patentamt

(19) **European Patent Office**

Office européen des brevets

(11) Veröffentlichungsnummer: **0 046 183**
**B1**

(12) · **EUROPÄISCHE PATENTSCHRIFT**

(45) Veröffentlichungstag der Patentschrift:
21.12.83

(51) Int. Cl.³: **C 07 D 201/12**

(21) Anmeldenummer: **81105375.0**

(22) Anmeldetag: **10.07.81**

(54) **Verfahren zur Gewinnung von Caprolactam durch Spaltung von Oligomeren des Caprolactams.**

(30) Priorität: **14.08.80 DE 3030735**

(43) Veröffentlichungstag der Anmeldung:
**24.02.82 Patentblatt 82/8**

(45) Bekanntmachung des Hinweises auf die Patenterteilung:
**21.12.83 Patentblatt 83/51**

(84) Benannte Vertragsstaaten:
**BE CH DE FR GB IT LI NL**

(56) Entgegenhaltungen:
**GB - A - 819 683**
**US - A - 3 939 153**

**CHEMICAL ABSTRACTS, Band 84, 1976, 26. Januar
1976, Seite 2, Nr. 17764j Columbus, Ohio, U.S.A. L.A.
DMITRIEVA et al.: "Catalytic depolyamidation of cyclic
oligomers of Y-caprolactam"
CHEMICAL ABSTRACTS, Band 63, Nr. 5, 1965, Spalte
5833e Columbus, Ohio, U.S.A.
CHEMICAL ABSTRACTS, Band 88, 1978, Nr. 24, 12. Juni
1978, Seite 18, Nr. 170762j Columbus, Ohio, U.S.A.
SOVIET INVENTIONS ILLUSTRATED, Section Chemical,
Derwent Publications, Ltd., July 1966, Section 1, P13**

(73) Patentinhaber: **BASF Aktiengesellschaft,
Carl-Bosch-Strasse 38, D-6700 Ludwigshafen (DE)**

(72) Erfinder: **Fuchs. Hugo, Dr., Egellstrasse 28,
D-6700 Ludwigshafen (DE)**
Erfinder: **Frommer, Elmar, Dr., Lisztstrasse 117,
D-6700 Ludwigshafen (DE)**
Erfinder: **Grosskinsky, Otto-Alfred, Dr.,
Semmelweisstrasse 8, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Gewinnung von Caprolactam durch Spaltung von Oligomeren des Caprolactams

Bei der Herstellung von Caprolactam erhält man zunächst Polycaprolactam mit einem Gehalt an Monomeren und Oligomeren von ca. 10%. Aus dem Monomeren enthaltenden Polycaprolactam werden die Monomeren und die Oligomeren nach bekannten Verfahren durch Wasser extrahiert. Nach Abdampfen des Wassers wird das extrahierte Caprolactam zurückgewonnen. Es verbleiben jedoch noch erhebliche Mengen an Oligomeren, die behandlungsbedürftig sind.

Aus der DE-PS 950 726 ist ein Verfahren zur Spaltung von Polycaprolactam in dessen Monomere bekannt, bei dem man Polycaprolactam mit Säuren wie Phosphorsäure unter Mitverwendung von Wasser erhitzt. Um ein qualitativ hochwertiges Caprolactam zu erhalten, ist es jedoch erforderlich, das so erhaltene Caprolactam mit Oxidationsmitteln zu behandeln. Dies ist jedoch technisch sehr aufwendig. Entsprechend der GB-PS 819 683 spaltet man Oligomere des Caprolactams indem man diese dampfförmig bei 400°C über eine Schicht aus inerten Materialien wie Sand oder Silikate leitet. Die erzielte Ausbeute beträgt jedoch nur 66%. Nach dem aus der SU-PS 176 680 bekannten Verfahren leitet man Oligomere des Caprolactams dampfförmig bei einer Temperatur von 250 bis 260°C durch eine katalytische Schicht von Aluminiumoxid. Dieses Verfahren ist ohne Schwierigkeiten nur dann anwendbar, sofern das monomere Lactam sowie die Oligomeren durch Dünnschichtverdampfung oder ähnliche Verfahren dem Polymeren entzogen und somit schon dampfförmig vorliegen. Das erhaltene Caprolactam hat zudem eine hohe Permanganattitrationszahl und UV-Kennzahl, so daß es nicht ohne weiteres mit Rohcaprolactam aufgearbeitet werden kann, ohne dessen Qualität erheblich zu vermindern.

Aus Chemical Abstracts, Band 84, 1976, Ref. 17764j, ist zu entnehmen, daß man mit Wasserdampf Cyclodimere des Caprolactams mittels Wasserdampf in Caprolactam spaltet.

Die erfindungsgemäß verwendeten Oligomeren enthalten nicht nur Cyclodimere des Caprolactams, sondern auch weitere Oligomere, z. B. bis zu einem Polymerisationsgrad von n = 9, d. h. auch Trimere, Tetramere und Pentamere. Dort ist nicht zu entnehmen, wie zu verfahren ist, um auch höhere Oligomere in Caprolactam zurückzuspalten, insbesondere wie die Spaltung von Oligomeren des Caprolactams zu gestalten ist, daß man hohe Ausbeuten erzielt und zugleich Caprolactam in einer Qualität erhält, die es erlaubt, dieses ohne Nachteil zusammen mit dem Rohcaprolactam aufzuarbeiten. Dies gilt insbesondere im Hinblick auf die Permanganattitrationszahl und die UV-Kennzahl.

Es ist deshalb erforderlich, so erhaltenes Caprolactam getrennt zu reinigen, was technisch sehr aufwendig ist.

Es war deshalb die technische Aufgabe gestellt, die Spaltung von Oligomeren des Caprolactams so zu gestalten, daß man hohe Ausbeuten erzielt und zugleich Caprolactam in einer Qualität erhält, die es erlaubt, dieses ohne Nachteile zusammen mit dem Rohcaprolactam aufzuarbeiten.

Diese Aufgabe wird gelöst in einem Verfahren zur Gewinnung von Caprolactam durch Spaltung von Oligomeren des Caprolactams, wobei man die Oligomeren bei erhöhter Temperatur in Gegenwart von Wasserdampf durch eine katalytische Schicht von Aluminiumoxid leitet, dadurch gekennzeichnet, daß man die Oligomeren flüssig oder in fester Form in ein Aluminiumoxidwirbelbett einbringt und unter Mitverwendung von 0,005 bis 10 Gew.-Teilen Wasser in Form von Wasserdampf je Gew.-Teil Oligomere bei einer Temperatur von 290 bis 400°C spaltet.

Das neue Verfahren hat den Vorteil, daß man neben hohen Ausbeuten zugleich Caprolactam mit einer verringerten Permanganattitrationszahl und UV-Kennzahl erhält. Das neue Verfahren hat auch den Vorteil, daß die Oligomeren vor der Spaltung nicht gesondert verdampft werden müssen. Darüber hinaus hat das neue Verfahren den Vorteil, daß das erhaltene Caprolactam ohne Qualitätsminderung mit Rohlactam verarbeitet werden kann.

Oligomere, die als Ausgangsstoffe eingesetzt werden, haben in der Regel einen Polymerisationsgrad n von 2 bis 9. Insbesondere enthalten sie dimere und trimere cyclische Oligomere. Solche Oligomere werden beispielsweise erhalten, durch Eindampfen von Waschwässern, die bei der Extraktion von Polycaprolactam anfallen und anschließende Entfernung des monomeren Caprolactam durch Destillation. Vorteilhaft wendet man die Oligomeren im Gemisch mit Caprolactam an. Es ist deshalb nicht erforderlich, aus dem anfallenden Extrakt die gesamte Menge Caprolactam abzudestillieren. Geeignete Gemische enthalten beispielsweise 10 bis 60 Gew.-% Oligomere und 90 bis 40 Gew.-% Caprolactam. Die Oligomeren oder die Gemische aus Oligomeren und Caprolactam werden vorteilhaft in flüssiger Form, d. h. in geschmolzenem Zustand, z. B. mit einer Temperatur von 150 bis 250°C in ein Aluminiumoxidwirbelbett eingebracht. Es ist jedoch auch möglich die Oligomeren bzw. das Oligomeren-Lactamgemisch in fester fein verteiler Form in die Wirbelschicht einzubringen und katalytisch in monomeres Caprolactam zu spalten. Das Einbringen in die Wirbelschicht erfolgt z. B. durch Einblasen mittels einer mit Inertgas betriebenen Düse. Als Aluminiumoxid eignen sich die verschiedenen Modifikationen, wie Tonerde, Böhmit. Besonders bewährt hat sich $\gamma$-Aluminiumoxid als Katalysator. Der Katalysator wird mit Inertgas, wie Kohlendioxid, Argon oder Stickstoff, vorzugsweise Stickstoff in wirbelnder Bewegung gehalten. Zweckmäßig verwendet man Aluminiumoxid in Korngrößen von 0,05 bis 1,5 mm, insbesondere von 0,2 bis 1 mm. Die Höhe der Katalysatorschicht wird vorteilhaft so gewählt, daß die Verweilzeiten der Oligomeren an der Katalysatorschicht von 0,1 bis 30, insbesondere von 0,5 bis 10 sek betragen. Vorteilhaft führt man das Verfahren bei Normaldruck durch. Es kann jedoch auch bei schwach vermindertem oder leichtem Überdruck, z. B. bis zu 2 bar durchgeführt werden.

Die Katalysatorschicht wird auf einer Temperatur von 290 bis 400°C, insbesondere 300 bis 360°C gehalten. Es ist deshalb auch zweckmäßig, das Inertgas mit einer Temperatur von 290 bis 400°C der Wirbelschicht zuzuführen.

Erfindungsgemäß führt man die Zersetzung unter Mitverwendung von Wasserdampf durch, hierbei verwendet man je Gew.-Teil Oligomere 0,005 bis 10 Gew.-Teile, insbesondere 0,02 bis 2 Gew.-Teile Wasser in Form von Wasserdampf. Das mitzuverwendende Wasser kann als solches in die Wirbelschicht eingebracht und dort verdampft werden. Vorzugsweise führt man das Wasser jedoch in Form von Wasserdampf zu. Beispielsweise kann dieser auch zusammen mit dem Inertgas in die Wirbelschicht eingeführt werden.

Das aus der Wirbelschicht austretende Gasgemisch wird, wie z. B. in DE-AS 1 445 549 beschrieben, in einer Glockenbodenkolonne durch Aufgabe von Wasser auf den Kopf der Kolonne kondensiert. Als Sumpfprodukt erhält man Caprolactam, über Kopf der Kolonne entweichen das Inertgas sowie Wasserdampf. Der Wasserdampf kann aus dem Inertgas kondensiert werden und das Inertgas zweckmäßig wieder in die Wirbelschicht zurückgeführt werden.

Das kondensierte Caprolactam kann z. B. durch Destillation nochmals gereinigt werden und anschließend das so zurückgewonnene Caprolactam dem zu reinigenden Caprolactam aus der Beckmannschen Umlagerung zugesetzt und zusammen, wie z. B. in DE-PS 1 194 863 oder in Ullmanns Encyklopädie der technischen Chemie, 4. Auflage, Band 9, Seite 100 beschrieben, aufgearbeitet werden.

Es ist aber auch möglich, das Caprolactam aus dem Reaktor austretenden Dämpfegemisch wie beschrieben zu kondensieren und dieses direkt dem Rohlactam aus der Beckmannschen Umlagerung zuzufügen und gemeinsam aufzuarbeiten.

Caprolactam wird für die Herstellung von Polycaprolactam verwendet. Das Verfahren nach der Erfindung sei an folgenden Beispielen veranschaulicht:

Zur Bestimmung der flüchtigen Basen (abgekürzt FBZ) werden in einer Kjeldahl-Apparatur 40 ml 30%ige Natronlauge und danach eine Lösung von 20 g Substanz in 80 ml destilliertes Wasser eingefüllt. Man spült mit 20 ml destilliertem Wasser nach. Dann destilliert man unter Einblasen von Wasserdampf in einer Zeit von 5 Minuten 50 ml Wasser in die Vorlage über, die 5 ml n/50 Salzsäure, 30 ml Wasser und 5 Tropfen Indikatorlösung enthält. Danach spült man den Kühler samt Ablaufrohr mit destilliertem Wasser in die Vorlage aus und titriert mit n/50 Natronlauge die nicht verbrauchte Säure zurück. Unter Berücksichtigung des Blindversuchs gibt der Verbrauch an n/50 Salzsäure unmittelbar die flüchtigen Basen in mäq Basen je kg Substanz.

Unter der »Permanganat-Titrationszahl«, abgekürzt »PTZ«, ist der Verbrauch an n/10 Kaliumpermanganatlösung in Millilitern zu verstehen, denn eine Lösung von 1000 g Substanz in 2500 g 50%iger wäßriger Schwefelsäure hat, bis bei Titration bei Raumtemperatur die Permanganatfarbe 2 Minuten beständig ist.

PAZ = Permanganat — Absorptionszahl

Gemessen wird die Extinktion aus der Lichtdurchlässigkeit einer 1prozentigen Caprolactamlösung in Wasser (50 ml bzw. 100 ml Lösung) nach Zugabe von 0,01 n KMnO₄-Lösung (1 bzw. 2 ml) bei 25°C gegen eine gleiche Lösung ohne Caprolactam nach 600 sec. bei 420 nm.

UV-Zahl

Prinzip

Im Spektralbereich von 360 bis 270 nm wird die Absorption des Caprolactams gemessen und nach Umsetzung in einer Kennzahl ausgedrückt.

Analysengeräte

1 registrierendes Einstrahlspektralphonometer
1 Erlenmeyerkolben (200 ml)
2 Quarz-Küvetten mit Deckel 10 cm lang (Schichtdicke 10 cm)

## 0 046 183

### Vorschrift

50 g Caprolactam werden in einem Erlenmeyerkolben in 50 g bidestilliertem Wasser kalt aufgelöst. Mit dieser Lösung wird eine Küvette bis zur Eichenmarke gefüllt. Die zweite Küvette wird mit dem gleichen bidestilliertem Wasser gefüllt und stellt die Vergleichslösung dar.

Nun werden beide Küvetten mit den Deckeln verschlossen, die geschliffenen Flächen mit Seidenpapier gereinigt und in die Küvettenhalter eingesetzt. Dann wird gemäß Geräteanleitung das Spektrum zwischen 370 nm und 260 nm aufgenommen. Die Registriergeschwindigkeit beträgt 50. Die Extinktionsmessung wird im Meßbereich 0 bis 1 ausgeführt.

Ist die Aufgabe beendet, wird von 270 bis 360 nm alle 10 nm eine Markierung auf dem Papier angebracht.

### Auswertung

Aus dem Diagramm werden die Extinktionen bei 270, 280, 290, 300, 310, 320, 330, 340, 350 und 360 nm abgelesen und addiert.

Die Summe der 10 Extinktionswerte wird mit 2 multipliziert und ergibt die UV-Kennzahl. Die UV-Kennzahl wird also immer auf 100%iges Caprolactam und auf eine Schichtdicke von 10 cm bezogen.

### Beispiel 1

In einem senkrecht stehenden, isolierten, elektrisch beheizten, unten mit einem Lochboden versehenen Rohr von 1200 mm Länge und 100 mm Durchmesser werden 1000 g Katalysator, bestehend aus einem bei 800°C geglühten $\gamma$-Aluminiumoxid der Korngröße 0,2 bis 0,8 mm, auf 320°C erhitzt. Durch einen von unten durch den Lochboden eingeblasenen auf 360°C vorgewärmten Stickstoffstrom von 2500 Nl/h wird der Katalysator zum Wirbeln gebracht. Durch eine 90 mm oberhalb des Lochbodens zentrisch im Rohr befindliche, nach oben gerichtete Zweistoffdüse wird mit Hilfe eines Stickstoffstromes von 1500 Nl/h der auf 200°C vorgeheizt wurde, aus einem auf 170°C warmen Vorratsgefäß im Laufe einer Stunde 4500 g einer Caprolactam-Oligomerenschmelze mit 30% Oligomeren in die Katalysatorschicht eingedüst. Dem vorgeheizten Stickstoffstrom werden stündlich 600 g Wasserdampf zugefügt. Die Temperatur im Katalysatorbett wird durch die elektrische Heizung bei 320°C gehalten. Die den Reaktor verlassenden Dämpfe werden in einer Glockenbodenkolonne vom Durchmesser 100 mm, mit 10 Böden durch Aufgabe von Wasser auf den Kolonnenkopf kondensiert. Man erhält so stündlich 4320 g Caprolactam, welches oligomerenfrei ist. Die Kennzahlen des erhaltenen Lactams bezogen auf wasserfreies Lactam sind folgende:

| | |
|---|---|
| PTZ | 180 |
| UV-Kennzahl | 520 |

500 g dieses Lactams werden zu 5000 g Extraktlactam mit folgenden Kennzahlen

| | |
|---|---|
| PTZ | 50 |
| UV-Kennzahl | 110 |
| fl. Basen | 0,6 mäq/kg |

gegeben und gemeinsam durch Destillation im Vakuum zu Reinlactam aufgearbeitet. Das erhaltene Reinlactam weist folgende Kennzahlen auf:

| | |
|---|---|
| Erstarrungspunkt | 69,1°C |
| PTZ | 1,7 |
| PAZ | 3,6 |
| UV-Kennzahl | 4,8 |
| Extinktion bei 290 nm in 1 cm Schichtdicke (50% wäßr. Lsg.) | 0,03 |
| fl. Basen | 0,15 mäq/kg |

### Vergleichsbeispiel 1

Wie in Beispiel 1 beschrieben, werden stündlich 4500 g einer Caprolactam-Oligomeren-Schmelze mit 30% Oligomeren mit einer Temperatur von 170°C ohne Mitverwendung von Wasserdampf in den Reaktor eingedüst. Als Katalysator dient $\gamma$-Aluminiumoxid der mit Hilfe von 2500 Nl/h Stickstoff der auf

4

0 046 183

360° C vorgeheizt wurde, zum Wirbeln gebracht wird. Das den Reaktor verlassende Dämpfegemisch wird in einer Glockenbodenkolonne wie beschrieben kondensiert. Auf diese Weise werden 4280 g Caprolactam mit folgenden Kennzahlen erhalten:

| | |
|---|---|
| PTZ | 1000 |
| UV-Kennzahlen | 1500 |

Im Vergleich zu Beispiel 1 sind die analytischen Werte des Spaltlactams erheblich schlechter.

### Vergleichsbeispiel 2

In einem Kolben versehen mit Thermometer, Gasableitungsrohr und Gaseinleitungsrohr werden 200 g Oligomere des Caprolactams vorgelegt. Die Oligomeren wurden aus einem Extraktionswasser der Polyamidherstellung durch Entfernen des Wassers und Abdestillieren des Lactams als Rückstand erhalten.

Nach Zugabe von 5% $H_3PO_4$ wurde der Kolbeninhalt mit Hilfe eines Metallbades auf 340° C bei Normaldruck erhitzt. Gleichzeitig wurden ca. 200 g Wasser verdampft, auf 340° C vorgeheizt und durch die Schmelze geleitet. Das aus dem Kolben austretende Dämpfegemisch wurde kondensiert und analysiert.

Es konnten auf diese Weise 175 g Caprolactam erhalten werden, welches folgende analytische Kennzahlen aufwies:

| | |
|---|---|
| PTZ | 2500 |
| UV-Kennzahl | 7500 |

Dieses Lactam wird in einer Menge von 1,5% bezogen auf 100%iges Lactam dem Extraktlactam mit folgenden Kennzahlen PTZ 50, UV-Kennzahl 110 flüchtige Basen 0,6 mäq/kg zugesetzt und durch Destillation wie beschrieben aufgearbeitet.

Das Reinlactam weist folgende Kennzahlen auf:

| | |
|---|---|
| PTZ | 6,0 |
| PAZ | 8,5 |
| UV-Kz | 15 |
| Extinktion bei 290 nm in 1 cm | |
| Schichtdicke 0,16 | 0,16 |
| Erstarrungspunkt | 69,0° C |
| flüchtige Basen | 0,48 mäq/kg |

Dieses Lactam ist somit außerhalb der üblichen Verkaufsspezifikationswerte.

### Beispiel 2

In einer Apparatur wie in Beispiel 1 beschrieben werden 1000 g $\gamma$-Aluminiumoxid als Katalysator auf 320° C erhitzt und mit 2500 Nl/h Stickstoff zum Wirbeln gebracht. Über eine Düse die mit ca. 2000 Nl/h Stickstoff betrieben wird, werden stündlich 400 g Oligomere in Pulverform in die heiße Katalysatorschicht gebracht. Mit dem zum Wirbeln notwendigen Stickstoff werden gleichzeitig 2000 g Wasserdampf in die Katalysatorschicht gegeben. Das den Reaktor verlassende Dämpfegemisch wird wie beschrieben kondensiert.

Stündlich werden 360 g monomeres Caprolactam mit folgenden Zahlen erhalten:

| | |
|---|---|
| PTZ | 300 |
| UV-Kennzahl | 400 |

250 g dieses Lactams werden mit 2500 g Extraktlactam zusammen wie beschrieben zu Reinlactam aufgearbeitet. Die Kennzahlen des Reinlactams sind folgende:

| | |
|---|---|
| Erstarrungspunkt | 69,12° C |
| PTZ | 1,4 |
| PAZ | 4,2 |
| UV-Kennzahl | 3,55 |
| Extinktion 290 nm in 1 cm | |
| Schichthöhe (50%ige wäßr. Lsg.) | 0,25 |
| fl. Basen | 0,11 mäq/kg |

5

Arbeitet man wie in Beispiel 2 beschrieben, fügt jedoch während der Spaltung keinen Wasserdampf hinzu, so erhält man bei einem Einsatz von 400 g Oligomeren pro Stunde 352 g monomeres Caprolactam mit folgenden Zahlen.

| PTZ | 1000 |
| UV-Kennzahl | 3000 |

Ein Zumischen dieses Lactams im gleichen Verhältnis wie in Beispiel 4 beschrieben zum Extraktlactam und anschließendes gemeinsames Aufarbeiten zum Reinlactam führt zu Kennzahlen die außerhalb der üblichen Verkaufsspezifikationswerte liegen.

## Patentansprüche

1. Verfahren zur Gewinnung von Caprolactam durch Spaltung von Oligomeren des Caprolactams, wobei man die Oligomeren bei erhöhter Temperatur in Gegenwart von Wasserdampf durch eine katalytische Schicht von Aluminiumoxid leitet, dadurch gekennzeichnet, daß man die Oligomeren flüssig oder in festem Zustand in eine Aluminiumoxidwirbelschicht einbringt und unter Mitverwendung von 0,005 bis 10 Gew.-Teilen Wasser in Form von Wasserdampf je Gew.-Teil Oligomere bei einer Temperatur von 290 bis 400° C spaltet.

2. Verfahren nach Anspruch 1, dadurch gekennzeichnet, daß man die Spaltung bei einer Temperatur von 300 bis 360° C durchführt.

3. Verfahren nach den Ansprüchen 1 bis 2, dadurch gekennzeichnet, daß man die Oligomeren zusammen mit Caprolactam in ein Aluminiumoxidwirbelbett einbringt.

4. Verfahren nach den Ansprüchen 1 bis 3, dadurch gekennzeichnet, daß man $\gamma$-Aluminiumoxid als Katalysator verwendet.

## Claims

1. A process for obtaining caprolactam by cleaving caprolactam oligomers by passing them through an alumina catalyst bed at an elevated temperature in the presence of steam, wherein the oligomers, as liquid or solid, are introduced into a fluidized bed of alumina and cleaved at from 290 to 400° C in the presence of from 0.005 to 10 parts by weight of water, in the form of steam, per part by weight of oligomer.

2. A process as claimed in claim 1, wherein the cleavage is carried out at from 300 to 360° C.

3. A process as claimed in claims 1 and 2, wherein the oligomers are introduced conjointly with caprolactam into a fluidized bed of alumina.

4. A process as claimed in claims 1 to 3, wherein the catalyst used is $\gamma$-alumina.

## Revendications

1. Procédé d'obtention de caprolactame par dédoublement d'oligomères du caprolactame, en faisant passer les oligomères, à température élevée, en présence de vapeur d'eau, à travers une couche catalytique d'oxyde d'aluminium, caractérisé par le fait qu'on introduit les oligomères, liquides ou à l'état solide, dans une couche d'oxyde d'aluminium en mouvement turbulent et qu'on les dédouble à une température de 290 à 400° C, en employant simultanément 0,005 à 10 parties en poids d'eau, sous forme de vapeur d'eau, par partie en poids d'oligomères.

2. Procédé selon la revendication 1, caractérisé par le fait qu'on effectue le dédoublement à une température de 300 à 360° C.

3. Procédé selon les revendications 1 et 2, caractérisé par le fait qu'on introduit les oligomères, en même temps que du caprolactame, dans un lit d'oxyde d'aluminium en mouvement turbulent.

4. Procédé selon l'une des revendications 1 à 3, caractérisé par le fait qu'on utilise de l'oxyde d'aluminium-$\gamma$ comme catalyseur.